# EUROPEAN PATENT SPECIFICATION

(11) **EP 1 634 893 B1**
(45) Date of publication and mention of the grant of the patent: **28.11.2007**
(21) Application number: 04104404.1
(22) Date of filing: 13.09.2004
(51) Int. Cl.: C08B 37/00

(54) **Process for the sulfation of chondroitin**
Verfahren zur Sulfierung von Chondroitin
Procédé de sulfatation de la chondroitine

(43) Date of publication of application: 15.03.2006
(73) Proprietor: LABORATORI DERIVATI ORGANICI S.P.A., 20122 Milano (IT)
(72) Inventor: Gonella, Sergio Dr., 12048, Santhià (IT); Bensi, Donata Dr., 13100, Vercelli (IT); Maggia, Giorgio Dr., 15033, Casale Monferrato (AL) (IT)
(74) Representative: Serravalle, Marco

(56) References cited:
- GB-A- 701 414
- PATENT ABSTRACTS OF JAPAN vol. 010, no. 208 (C-361), 22 July 1986 (1986-07-22) & JP 61 047701 A (SEIKAGAKU KOGYO CO LTD), 8 March 1986 (1986-03-08) & JP 61 047701 A (SEIKAGAKU KOGYO CO LTD) 8 March 1986 (1986-03-08)

## Description

The present invention is directed to a one-step process for the sulfation and depolymerization of chondroitin.

Chondroitin is a natural product characterized by high molecular weight (around 50.000 Da) and low degree of sulfation (6-7 % organic sulfur). Depolymerized chondroitin sulfate derivatives having organic sulfur content of about 25 to 37 % are useful in the treatment of thrombophlebitis, inflammation of varix, traumatic disorders, edema etc..

US 4,524,066 discloses process for the preparation of low molecular weight polysulphated chondroitin according to the following steps: oxidative depolymerization of a crude chondroitin polysulfate corresponding to the prior art, if necessary bleaching with peracetic acid, isolation of a fraction with a mean molecular weight 5,000-15,000 by precipitation with methanol and/or ultrafiltration, if necessary demineralization by means of ion exchangers and if necessary treatment with active charcoal.

The present invention discloses a single step process which allows the preparation of chondroitin polysulfate with intrinsic viscosity comprised between 0.09 and 0.18 dl/g, a high tenor of sulfur and good purity characteristics. The process comprises dissolving the natural chondroitin in formamide and adding chlorosulfonic acid to the solution kept refrigerated and under stirring, at a rate that allows the temperature in the reactor to remain lower than 40 °C and higher than 15 °C, preferably from 20° to 35°C, more preferably from 20° to 30 °C.

Since the reaction is considerably exotermic, the addition rate depends on the dimension of the batch (volume of reaction solution) and on the temperature of the refrigerating liquid. In fact, the larger is the volume of the solution, the longer is the addition time, since removal of the reaction heat becomes comparatively slower.

When the addition is too fast and the temperature rises higher than 40 °C, the sulfation reaction is not efficient resulting in a chondroitin with a Hyaluronidase inhibiting effect lower than 14%.

If the temperature of the solution is kept equal to or lower than 15 °C, the sulfation of chondroitin takes place without significant depolymerization, resulting in a chondroitin polysulfate having intrinsic viscosity higher than 0.18 dl/g.

The concentration of chondroitin in formamide can vary in a broad range, but it is preferably between 5 and 20 %. If the concentration is lower than 5 %, the reaction volumes grow too much, if it is higher than 20%, it is more difficult to remove the reaction heat.

Chondroitin polysulfate is a product of pharmaceutical interest and it is commercialized under the name "Heparinoid" or HPA (heparin analogue). The commercial product is characterized by the following analytical data:

| | |
|---|---|
| Organic sulfur | 25.8% - 37.3% |
| Uronic acid | 19% - 24% |
| Intrinsic Viscosity | 0.09 - 0.18 dl/g |
| Hyal. Inh. effect | 14 - 29% |
| Optical Rotation | - 11.7° / -14.7° |

In a preferred embodiment of the invention, the process is conducted in such a way to produce a chondroitin polysulfate in accordance with the specifications of Heparinoid.

### Examples

Organic sulfur, Uronic acid, intrinsic viscosity, Hyaluronidase inhibiting effect and
Optical rotation were measured in accordance with the Japanese Pharmaceutical codex 1997

### Example 1

601 of a 10 % w/v solution of chondroitin in formamide were placed in a reactor under stirring. The reactor is cooled down by water circulating in the double jacket. 5 1 of chlorosulfonic acid were added at a rate of 18.5 ml/min. The temperature of the solution during addition remained around 25 °C. After 4h 30' the addition was completed. The analytical values of the obtained chondroitin polysulfate are listed in Table 1.

### Example 2

600 ml of a 10 % w/v solution of chondroitin sulfate in formamide were placed in a reactor under stirring. The reactor is cooled down by water circulating in the double jacket. 50 ml of chlorosulfonic acid were added at a rate of 18.5 ml/min. The temperature of the solution during addition remained around 25 °C. After 3' the addition was completed.
The analytical values of the obtained chondroitin polysulfate are listed in Table 1.

### Comparative Example 3

Example 2 was repeated but at a lower addition rate of chlorosulfonic acid (0.2 ml/min). The addition time was 4h 10' and the temperature of the solution was 15 °C.
The analytical values of the obtained chondroitin polysulfate are listed in Table 1.

### Comparative Example 4

Example 1 was repeated but increasing the amount of chlorosulfonic acid (7.51) and the temperature in the reactor is thermostated at 40 °C.
The analytical values of the obtained chondroitin polysulfate are listed in Table 1.

### Comparative Example 5

Example 1 was repeated but increasing the rate of addition of the chlorosulfonic acid (25 ml/min) and the temperature in the reactor is thermostated at 40 °C.
The analytical values of the obtained chondroitin polysulfate are listed in Table 1.

After the reaction with chlorosulfonic acid, all the solutions (exemples 1-5) were treated with acetone, ion exchange resins, pH adjustment, under vacuum distillation and dried to obtain the powder below analysed

**Table 1**

| Analysis | Specifications | Ex. 1 | Ex. 2 | C. Ex. 3 | C. Ex. 4 | C. Ex. 5 |
|---|---|---|---|---|---|---|
| Organic Sulfur | 25.8-37.3 % | 28.35% | 27.3% | 27.9% | 41.4% | 34.16% |
| Uronic acid | 19 ― 24 % | 19.25% | 22.9% | 23.9% | 20.69% | 21.60% |
| Intrinsic Viscosity | 0.09 ― 0.18 | 0.17 | 0.17 | 0.24 | 0.17 | 0.11 |
| Hyalur. Inhib. effect | 14 ― 29% | 20.8% | 18.5% | 9.5% | 16.35% | 10.44% |
| Optical Rotation | -11.7/-14.7° | -12.31° | - 13.2° | - 15.2° | - 12.31° | - 13.63° |

## Claims

1. Process for the preparation of chondroitin polysulfate with intrinsic viscosity comprised between 0.09 and 0.18 and organic sulfur content comprised between 25.8 and 37.3 % as measured in accordance with the Japanese Pharmaceutical codex 1997, **characterized in that** chlorosulfonic acid is added to a formamide solution of chondroitin at a rate such that the temperature of the formamide solution is kept lower than 40 °C and higher than 15°C.

2. Process according to claim 1 wherein the temperature of the formamide solution is kept in the range 20-35 °C.

3. Process according to claim 1 wherein the temperature of the formamide solution is kept in the range 20-30 °C.

4. Process according to claims 1-3 wherein the concentration of chondroitin sulphate in formamide is comprised between 5 and 20 % w/v.

5. Process according to claims 1-4 wherein the activity of the obtained chondroitin polysulfate in the inhibition of Hyaluronidase is comprised between 14 and 29 as measured in accordance with the Japanese Pharmaceutical codex 1997.

## Patentansprüche

1. Verfahren zur Herstellung von Chondroitinpolysulfat mit einer intrinsischen Viskosität zwischen 0,09 und 0,18 und mit einem organischen Schwefelgehalt zwischen 25,8 und 37,3%, gemessen in Übereinstimmung mit dem Japanese Pharmaceutical Codex 1997, **dadurch gekennzeichnet, dass** Chlorsulfonsäure zu einer Formamidlösung von Chondroitin in solchem Maße zugegeben wird, dass die Temperatur der Formamidlösung unterhalb von 40°C und oberhalb von 15°C gehalten wird.

2. Verfahren nach Anspruch 1, wobei die Temperatur der Formamidlösung im Bereich von 20-35°C gehalten wird.

3. Verfahren nach Anspruch 1, wobei die Temperatur der Formamidlösung im Bereich von 20-30°C gehalten wird.

4. Verfahren nach den Ansprüchen 1-3, wobei die Konzentration von Chondroitinsulfat in Formamid zwischen 5 und 20 Gew./Vol.-% liegt.

5. Verfahren nach den Ansprüchen 1-4, wobei die Aktivität des erhaltenen Chondroitinpolysulfats bei der Hemmung von Hyaluronidase zwischen 14 und 29 liegt, gemessen in Übereinstimmung mit dem Japanese Pharmaceutical Codex 1997.

## Revendications

1. - Procédé de préparation de polysulfate de chondroïtine ayant une viscosité intrinsèque comprise entre 0,09 et 0,18 et une teneur en soufre organique comprise entre 25,8 et 37,3% telle que mesurée conformément au Codex Pharmaceutique Japonais 1997, **caractérisé par le fait que** de l'acide chlorosulfonique est ajouté à une solution de chondroïtine dans le formamide à une vitesse telle que la température de la solution de formamide est maintenue inférieure à 40°C et supérieure à 15°C.

2. - Procédé selon la revendication 1, dans lequel la température de la solution de formamide est maintenue dans la plage de 20-35°C.

3. - Procédé selon la revendication 1, dans lequel la température de la solution de formamide est maintenue dans la plage de 20-30°C.

4. - Procédé selon l'une des revendications 1 à 3, dans lequel la concentration du sulfate de chondroïtine dans le formamide est comprise entre 5 et 20% p/v.

5. - Procédé selon l'une des revendications 1 à 4, dans lequel l'activité du polysulfate de chondroïtine obtenu dans l'inhibition de la Hyaluronidase est comprise entre 14 et 29 telle que mesurée conformément au Codex Pharmaceutique Japonais 1997.
